# EUROPEAN PATENT SPECIFICATION

(11) **EP 2 521 732 B1**
(45) Date of publication and mention of the grant of the patent: **06.07.2016**
(21) Application number: 11700029.9
(22) Date of filing: 04.01.2011
(51) Int. Cl.: A61K 38/46, A61K 31/11, A61K 31/69, C07K 5/083, C11D 3/16, C11D 3/20, C11D 3/26, C11D 3/386, C07K 5/09, C07K 5/11, C11D 3/04, C11D 3/36, C07K 5/107, A61K 45/06, A61K 31/165

(54) **Lipase formulation**
Lipaseformulierung
Formulation de lipase

(30) Priority: 08.01.2010 EP 10150333
(43) Date of publication of application: 14.11.2012
(73) Proprietor: Novozymes A/S, 2880 Bagsvaerd (DK)
(72) Inventor: MIKKELSEN, Lise, Munch, DK-2610 Roedovre (DK); HANSEN, Tomas, Tage, DK-3450 Alleroed (DK); FRIIS, Esben, Peter, DK-2730 Herlev (DK)
(86) International application number: PCT/EP2011/050065
(87) International publication number: WO 2011/083114

(56) References cited:
- WO-A1-94/29428
- WO-A1-2009/068098
- WO-A1-2009/102854
- WO-A2-2009/118375
- GB-A- 2 091 270
- RUCHI G ET AL: "Lipase from solvent tolerant Pseudomonas aeruginosa strain: Production optimization by response surface methodology and application", BIORESOURCE TECHNOLOGY, ELSEVIER BV, GB LNKD- DOI:10.1016/J.BIORTECH.2007.09.053, vol. 99, no. 11, 1 July 2008 (2008-07-01), pages 4796-4802, XP022606287, ISSN: 0960-8524 [retrieved on 2007-10-31]
- KASCHANI FARNUSCH ET AL: "Diversity of serine hydrolase activities of unchallenged and botrytis-infected Arabidopsis thaliana", MOLECULAR AND CELLULAR PROTEOMICS, AMERICAN SOCIETY FOR BIOCHEMISTRY AND MOLECULAR BIOLOGY, INC, US, vol. 8, no. 5, 1 May 2009 (2009-05-01), pages 1082-1093, XP009128980, ISSN: 1535-9484

## Description

### FIELD OF THE INVENTION

The present invention relates to a composition which comprises a lipase and which does not comprise a protease.

### BACKGROUND OF THE INVENTION

Lipases for industrial use may be supplied in a liquid or granule formulation for use, e.g., in liquid or granular detergents. The formulation is typically free of protease and/or free of surfactant, and it is generally desirable to improve the storage stability of the lipase in the formulation.

WO 2009/118375 discloses that in a liquid detergent containing a protease and a lipase, the storage stability of the lipase can be improved by the addition of a peptide aldehyde due to inhibition of the protease.

### SUMMARY OF THE INVENTION

The inventors have found that the storage stability of a lipase in a formulation without a protease can be improved by incorporating a serine protease inhibitor. Without wishing to be bound by theory, the inventors believe that the structure of the lipase is stabilized by incorporating a serine protease inhibitor in the active site.

Accordingly, the invention provides a composition which comprises a serine hydrolase acting on ester bonds which is a lipase and a serine protease inhibitor, and which does not comprise a protease as defined in the claims.

### DETAILED DESCRIPTION OF THE INVENTION

### Lipase

The serine hydrolase acting on ester bonds which is a belongs to enzyme class EC 3.1.1.3.

The lipase may be *Thermomyces lanuginosus* lipase (TLL, shown as SEQ ID NO: 2 in WO 2009/109500), Candida *antarctica* lipase A (CALA, shown as SEQ ID NO: 1 in WO 2008/040739), *Candida antarctica* lipase B (CALB, having the amino acid sequence described in J. Uppenberg et al., Structure, 1994, 2:293-308), *Rhizomucor miehei* lipase, *Aspergillus niger* lipase, *Candida rugosa* lipase (SWISSPROT:P20261), *Penicillium roqueforti* lipase, *Rhizopus niveus* lipase (SWISPROT:P61872), *Rhizopus oryzae* lipase, *Alcaligenes sp.* lipase, *Achromo-bacter sp.* lipase, *Burkholderia cepacia* lipase, *Pseudomonas stutzeri* lipase, or it may be a variant which has an amino sequence with at least 80% identity to one of these, particularly at least 85%, at least 90%, at least 95% or at least 98% identity.

Examples of TLL homologues are described in WO 1992/005249 (NZ 3520, Lipolase Ultra), WO0060063, WO9707202, WO0032758, WO02055679, WO04099400, WO07087508 and WO 2009/109500. Commercial lipases include the following products of Novozymes A/S: Novozym™ 435, Novozym 735, Lipozyme™ RM, Novozym 388, Lipolase Ultra™, Lipex™, Lipo-prime™, Resinase™ HT, Lipolase™, Resinase™, Lipopan™, Lipoclean™ and Lipolex™. Commercial lipases also include the following products of Amano Enzyme, Danisco, DSM, Meito Sangyo: Lipase A, LipaseG, Lipase AY, Lipase R, Newlase™ F, Grindamyl™, Powerbake™, BakeZyme L, BakeZyme PH, Panamore, Panamore Golden, Panamore Spring, Lipase MY, Lipase OF, Lipase PL, Lipase QL, Lipase AL, Lipase SL, Lipase TL, and Lipase UL.

### Serine protease inhibitor

The serine protease inhibitor is selected from inhibitors to proteases from the serine hydrolase superfamily. In particular, the serine protease inhibitor may be a peptide aldehyde or a boronic acid derivative.

The inhibitor may have an inhibition constant to a serine protease Ki (M, mol/L) of 1 E-12 - 1E-03; more preferred 1E-11 - 1E-04; even more preferred: 1E-10 - 1E-05; even more preferred 1E-10 - 1E-06; most preferred 1E-09 - 1E-07.

The serine protease inhibitor has the general formula A⁴-A³-A²-A¹, where
- A¹ is an aldehyde (CHO) or aldehyde analogue (COCX₃, COCHX₂, COCH₂X or COCH₃, where X is a halogen atom), a phosphorous derivative or a boronic acid (B(OH)₂) with a possibility to bind to the serine in the active site of the serine hydrolase.
- A² may be an aromatic, or an aliphatic part, which might be an optionally substituted phenyl, an aliphatic chain with at least 2 carbons (optionally branched with aliphatic or aromatic substituents), or an amino acid residue with aromatic or aliphatic side chain. If A² is an amino acid, then the C-terminal carbon is included in A¹ described above. Preferred amino acids are D- or L-Tyr (p-tyrosine), m-tyrosine, 3,4-dihydroxy-phenylalanine, Leu, Phe, Val, Met, Nva, Nle and preferred phenyl derivatives are phenyl, 2-, 3- or 4-formylphenyl. Preferred aliphatic derivatives are-CH2-CH(R")- with R"= H, CH₃, CH₂Ph, CH(CH₃)₂, CH₂C₆H₄-pOH and other amino acid side chain analogues). When A¹ is a boronic acid, A² may also be OH yielding boric acid/borax.
- A³ if present may a non-bulky linker, preferably hydrophobic, consisting of either 1-3 small amino acids (ex Gly, Ala, Leu, norleucine, norvaline) or a substituted/unsubstituted aliphatic chain with 1-12 atoms, preferably carbon, oxygen and nitrogen, most preferred 4-10 atoms.
- A⁴ if present may be a protection group (e.g. an N-capping group as described below), any amino acid, a small or a bulky group.

The serine protease inhibitor may be a peptide aldehyde or an aldehyde analogue having the formula B²-B¹-B⁰-R wherein:
a) R is hydrogen, CH₃, CX₃, CHX₂, or CH₂X, wherein X is a halogen atom; a phosphonate or a boronic acid (B(OH)2)..
b) B⁰ is a single amino acid residue;
c) B¹ is 1-2 amino acid residues; and
d) B² consists of one or more amino acid residues (preferably one or two), optionally
comprising an N-terminal protection group or an N-terminal protection group attached to B¹ (preferred if B¹ is a dipeptide).

In the above formula, B⁰ may be an L or D-amino acid with an optionally substituted aliphatic or aromatic side chain, preferably D- or L-Tyr (p-tyrosine), m-tyrosine, 3,4-dihydroxyphenylalanine, Leu, Phe, Val, Gly, Met, Trp, Ile, Nva or Nle. If R is boronic acid or a phosphonate then B⁰ is an amino acid residue without double-bound oxygen on the C-terminal carbon.

B¹ may be 1-2 amino acid residues, preferably 2, with a small optionally substituted aliphatic side chain, preferably selected from Ala, Cys, Gly, Leu, Arg, Pro, Ser, Ile, Thr, Val, Nva, or Nle, most preferred selected from Ala, Gly, Leu, Nva or Nle. The most preferred combinations are, Gly-Ala, Ala-Ala, Gly-Gly, and Gly-Leu.

B² may be an N-capping group known from protein chemistry, preferably selected from benzyloxycarbonyl (Cbz), p-methoxybenzyl carbonyl (MOZ), benzyl (Bn), benzoyl (Bz), p-methoxybenzyl (PMB), p-methoxyphenyl (PMP), formyl, acetyl, methyloxy, methyloxycarbonyl/methyl carbamate, or methyl urea. or B2 may be any1-2 amino acid residues without specifying structure optionally comprising a protection group as described above.

More particularly, the peptide aldehyde may be Z-GGY-H, Z-GGF-H, Z-GGG-H, Z-GAG-H, Z-RAY-H, Ac-GAY-H, Z-GAY-H, Z-GAL-H, Z-GAF-H, Z-GAV-H, Z-RVY-H, Z-LVY-H, Ac-LGAY-H, Ac-FGAY-H, Ac-YGAY-H, Ac-FGVY-H, Ac-FGAM-H, Ac-WLVY-H, MeO-CO-VAL-H, MeO-CO-LLY-H, MeOCO-FGAL-H, MeO-FGAF-H, MeNCO-FGAL-H Z-VAL-CF₃, Ac-NHCH(CH₂CH(CH₃))-B(OH)₂, Ac-NHCH(CH₂Ph)-B(OH)₂, MeOCO-FGA-NHCH(Ph)CH₂-B(OH)₂, Z-GA-N(CH₃)CH(Ph)CH₂-B(OH)₂- wherein Z is benzyloxycarbonyl, Me is methyl and Ac is acetyl.

Alternatively, the serine protease inhibitor may be a peptide aldehyde with the formula:

P-O-(Aⁱ-X')ₙ-Aⁿ⁺¹-Q

wherein Q is hydrogen, CH₃, CX₃, CHX₂, or CH₂X, wherein X is a halogen atom;
wherein one X' is the "double N-capping group" CO, CO-CO, CS, CS-CS or CS-CO,
most preferred ureido/"urea" (CO), and the other X' es are nothing,
wherein n = 1-10, preferably 2-5, most preferably 2,
wherein each of Aⁱ and Aⁿ⁺¹ is an amino acid residue having the structure:
   -NH-CR-CO- for a residue to the right of X= -CO-, or
   -CO-CR-NH- for a residue to the left of X= -CO-
wherein R is H- or an optionally substituted alkyl or alkylaryl group which may optionally include a hetero atom and may optionally be linked to the N atom. Preferred amino acids at the C-terminal (Aⁿ⁺¹) are L or D-amino acids with an optionally substituted aliphatic or aromatic side chain, preferably D- or L-Tyr (p-tyrosine), m-tyrosine, 3,4-dihydroxyphenylalanine, Leu, Phe, Val, Gly, Met, Trp, Ile, Nva or Nle. Preferred amino acid residues Ai (i ≤ n) to the right of X= -CO are those with small optionally substituted aliphatic side chain, preferably selected from Ala, Cys, Gly, Leu, Arg, Pro, Ser, Ile, Thr, Val, Nva, or Nle,
wherein P is hydrogen or any C-terminal protection group.

Examples of such peptide aldehydes include α-MAPI, β-MAPI, F-urea-RVY-H, F-urea-GGY-H, F-urea-GGF-H, F-urea-GAF-H, F-urea-GAY-H, F-urea-GAL-H, F-urea-GA-Nva-H, F-urea-GA-Nle-H, Y-urea-RVY-H, Y-urea-GAY-H, F-CS-RVF-H, F-CS-RVY-H, F-CS-GAY-H, Antipain, GE20372A, GE20372B, Chymostatin A, Chymostatin B, and Chymostatin C. Further examples of peptide aldehydes are disclosed in WO 2009/118375 and PCT/EP2009/053580, WO 94/04651, WO 98/13459, WO 98/13461, WO 98/13462, WO 2007/145963, (P&G) hereby incorporated by reference.

As another alternative, the serine protease inhibitor may be a borate such as borax. It may also be a boronic acid derivative having the formula B(OH)₂-C₆H₄-R wherein -C₆H₄-has bonds attached in the m- or p-position, and R is selected from the group consisting of CHO, , COOH, C₁-C₆ alkyl substituted CO-C₁-C₆ alkyl, C₁-C₆ alkenyl and substituted C₁-C₆ alkenyl, X (halogen),; most preferred is 4-formyl phenylboronic acid (4-FPBA) and borax.

As another alternative the serine protease inhibitor may be an aliphatic aldehyde, phosphonate or boronic acid with the structure:
D²-D¹-W
   - W is an aldehyde (CHO) or aldehyde analogue (COCX₃, COCHX₂, COCH₂X or COCH₃, where X is a halogen atom), a phosphorous derivative or a boronic acid (B(OH)2).
   - D¹ may be a hydrophobic chain of at 5-15 atoms, preferably carbon, oxygen and nitrogen, most preferred 6-8 atoms, if branched at position 3-8 (counting from W), then it must be with small substituents, preferably hydrophobic. If branched at position 1 or 2 (counting from W) then it must be an optionally substituted phenyl or aliphatic chain.
   - D² if present, may be H, a protection group, any amino acid, a small or a bulky group.

Examples of such inhibitors are Z-NH-(CH₂)₇-CHO, Z-NH-(CH₂)₆-CH(CH₂Ph)-CHO and Z-NH-(CH₂)₆-CH(CH₂C₆H₄-pOH)-CHO.

Further examples of inhibitors are described in WO 95/25791, WO9813459, WO9813461, WO9813462, WO2007145963 and WO2007141736.

### Lipase composition

The composition comprises a lipase and a serine protease inhibitor, and it does not contain a protease. It may be in granular or liquid form. It may be a detergent, or a non-detergent, i.e. a lipase formulation without a surfactant.

### Non-detergent composition

For a non-detergent composition, the lipase is present in an amount of 5-40 mg of enzyme protein per g, more preferred 10-30 mg enzyme protein/g. If the serine protease inhibitor is a peptide aldehyde or analogue it may be present in an amount of 0.01%-1%, more preferred 0.02%-0.5% or at a molar ratio of inhibitor:lipase of 0.5:1 to 20:1, more preferred 1:10 and even more preferred 1:5. If the serine protease inhibitor is a boric acid derivative (ex borax or substituted/unsubstituted phenylboronic acid derivatives) the inhibitor is present in an amount of 0.5-20%; more preferred 1%-10%.

Other ingredients in a non-detergent liquid compositions may be - but not limited to - polyols or mixtures of polyols (ex sorbitol, glycerol, monopropylene glycol, etc.)in 1-60%, small amounts of non-ionic surfactants (ex softanol) 0-5%, calcium ions 0.001%-0.5%, water, and preservatives (ex proxel, 2-phenoxyethanol, etc).

The serine protease inhibitor may be used to stabilized a lipase in a particle for triggered release of a rinse benefit agent comprising a lipase substrate, e.g. as described in WO2009/118329.

### Detergent composition

For a liquid or granular detergent, the non-detergent composition described above may be added in an amount of 0.01%-5%, more preferred 0.1-2.5%, or the enzyme and the protease inhibitor may be added separately. The lipase may be present in an amount of 0.1-2 mg enzyme protein per g detergent. If the serine protease inhibitor is a peptide aldehyde or analogue it may be present in an amount of 0.01 ppm-0.02%, more preferred 0.5 ppm-0.01%, even more preferred 0.8 ppm-40 ppm, or at a molar ratio of inhibitor:lipase of 0.5:1 to 20:1, more preferred up to 10:1 and even more preferred up to 5:1. If the serine protease inhibitor is a boric acid derivative (ex borax or substituted/unsubstituted phenylboronic acid derivatives) the inhibitor is present in an amount of 0.1 ppm-3%, more preferred 1 ppm-1%, even more preferred 5 ppm-0.5%, most preferred 30 ppm-0.2%. Other ingredients may include chelating agents, solvents, colorants, and perfumes. If the detergent is a liquid, then pH is between 5-10, more preferred between 7-10 or 7-9.

The liquid detergent may be aqueous, typically containing 5-95% water, e.g. 5-15% or 20-70% water and 0-20% organic solvent (hereinafter, percentages by weight).

The detergent comprises a surfactant which may be anionic, non-ionic, cationic, amphoteric or a mixture of these types. The detergent will usually contain 5-30% anionic surfactant such as linear alkyl benzene sulphonate (LAS), alpha-olefin sulphonate (AOS), alcohol ethoxy sulphate (AES) or soap. It may also contain 3-20% anionic surfactant such as nonyl phenol ethoxylate or alcohol ethoxylate.

The pH (measured in aqueous detergent solution) will usually be neutral or alkaline, e.g.. The detergent may contain 1-40% of a detergent builder such as zeolite, phosphate, phosphonate, citrate, NTA, MGDA, GLDA, EDTA or DTPA, or it may be unbuilt (i.e. essentially free of a detergent builder). It may also contain other conventional detergent ingredients, e.g. fabric conditioners, foam boosters, bactericides, optical brighteners and perfumes.

The detergent composition may be a fabric cleaning compositions, hard surface cleansing compositions, light duty cleaning compositions including dish cleansing compositions and automatic dishwasher detergent compositions.

The liquid detergent composition may comprise from about 0.0001 % to about 10%, more particularly from about 0.00015% to about 1%, and most particularly from about 0.001% to about 0.1% of the inhibitor

Thus, a stabilized liquid enzyme formulation typically contains 0.5-20% by weight, particularily 1-10% by weight, of enzyme protein (total of protease and optional second enzyme) and 0.01%-10% of the inhibitor, more particularly 0.05-5% by weight and most particularly 0.1%-2% by weight of the inhibitor.

A liquid detergent formulation will typically contain 0.04-400 micromolar enzyme (or 1-10000 mg EP/L), more particularily 0.16-160 micromolar enzyme (4-4400 mg EP/L) and most particularly 0.8-80 (20-2200 mg EP/L) and about 1-20 times more of the inhibitor, most particularly about 1-10 times more of the inhibitor.

The liquid detergent composition may contain water and other solvents as carriers. Low molecular weight primary or secondary alcohols exemplified by methanol, ethanol, propanol, and iso-propanol are suitable. Monohydric alcohols are preferred for solubilizing surfactants, but polyols such as those containing from about 2 to about 6 carbon atoms and from about 2 to about 6 hydroxy groups (e.g., 1,3-propanediol, ethylene glycol, glycerol, and 1,2-propanediol) can also be used. The compositions may contain from about 5-90%, 10-50% 2-50%; 2-25% of such carriers.

The detergent compositions herein will preferably be formulated such that during use in aqueous cleaning operations, the wash water will have a pH between about 6.8 and about 11. Finished products are typically formulated at this range. Techniques for controlling pH at recommended usage levels include the use of, for example, buffers, alkalis, and acids. Such techniques are well known to those skilled in the art.

When formulating the hard surface cleaning compositions and fabric cleaning compositions of the present invention, the formulator may wish to employ various builders at levels from about 5% to about 50% by weight. Typical builders include the 1-10 micron zeolites, polycarboxylates such as citrate and oxydisuccinates, layered silicates, phosphates, and the like. Other conventional builders are listed in standard formularies. The invention is particularly applicable to the formulation of liquid detergents where enzyme stability problems are pronounced.

The detergent may in particular be formulated for laundry, dishwashing or hard surface cleaning, e.g. for floor cleaning, carwash, industrial and institutional laundry, or washing of wool and silk textiles. The detergent may be formulated as described in US2006247150, WO2005040320, US2005020466, US2009217463, WO2008010920, WO2007087259, WO9600277, WO9511292, EP430315, DE10023580, WO9617052, WO2009107091, WO2008010920, WO2007087259, WO9600277, JP2006131659, JP2004204084 JP2004203918, WO2009060966, WO2006050308 and JP63260997.

### Use of lipase

The serine protease inhibitor may be used to stabilize the lipase in the absence of a protease. Thus, the inhibitor may be used in the manufacture of the lipase, e.g. by adding it during fermentation, recovery or during formulation of the lipase, e.g. as described in WO95/25791.

### EXAMPLES

### Example 1: Liquid lipase formulation

### Lipoclean formulations, per se (LCLU/g) residual activities:

The Lipase was formulated from crude concentrate with standard formulation ingredients (sorbitol and glycerol) with and without the serine protease inhibitor (2% enzyme protein, 0.08% Z-GAY-H added with a 1% DMSO solution). The solution was split into separate vials which were incubated at different temperature and times and compared in activity with a frozen reference sample frozen directly after mixing. The lipase activity was measured by ester bond hydrolysis in the substrate PNP-Palmitate (C:16) and releases PNP which is yellow and can be detected at 405 nm.

| | 2 weeks, 50°C | 4 weeks, 40°C | 4 weeks, 50°C |
|---|---|---|---|
| No inhibitor | 54% | 45% | 40% |
| 0,08% serine protease inhibitor | 71% | 70% | 56% |

## Claims

1. A composition which comprises a serine hydrolase acting on ester bonds which is a lipase and a serine protease inhibitor, and which does not comprise a protease, the composition being either a detergent composition or a non-detergent composition, wherein the non-detergent composition comprises the said lipase in an amount of 5-40 mg of enzyme protein per g.

2. The composition of claim 1 which is in the form of granules or a liquid.

3. The composition of claim 1 or 2 which is a detergent composition further comprising a surfactant.

4. The composition of claim 1 or 2 which does not comprise a surfactant.

5. The composition of any preceding claim wherein the lipase has an amino acid sequence which is at least 80% identical to *Thermomyces lanuginosus* lipase, *Candida antarctica* lipase A or *Candida antarctica* lipase B.

6. The composition of any preceding claim wherein the serine protease inhibitor has the general formula:
A⁴-A³-A²-A¹,
where
A¹ is an aldehyde (CHO) or aldehyde analogue (COCX₃, COCHX₂, COCH₂X or COCH₃, where
X is a halogen atom), a phosphorous derivative or a boronic acid (B(OH)₂);
A² may be an aromatic, or an aliphatic part, if A² is an amino acid, then the C-terminal carbon is included in A¹ described above;
A³ if present may a non-bulky linker, preferably hydrophobic;
A⁴ if present may be a protection group (e.g. an N-capping group as described below), any amino acid, a small or a bulky group.

7. The composition of any preceding claim wherein the serine protease inhibitor is a peptide aldehyde.

8. The composition of any preceding claim wherein the peptide aldehyde has the formula
B²-B¹-B⁰-R
wherein:
R is hydrogen, CH₃, CX₃, CHX₂, or CH₂X, wherein X is a halogen atom;
B⁰ is a single amino acid residue;
B¹ is a single amino acid residue; and
B² consists of one or more amino acid residues (preferably one or two), optionally comprising an N-terminal protection group.

9. The composition of any of claims 1-5 where the serine protease inhibitor has the formula:
P-O-(Aⁱ-X')ₙ-Aⁿ⁺¹-O
wherein Q is hydrogen, CH₃, CX₃, CHX₂, or CH₂X, wherein X is a halogen atom;
wherein one X' is the "double N-capping group" CO, CO-CO, CS, CS-CS or CS-CO, most preferred ureido/"urea" (CO), and the other X'es are nothing;
wherein n = 1-10, preferably 2-5, most preferably 2;
wherein each of Aⁱ and Aⁿ⁺¹ is an amino acid residue having the structure:
-NH-CR-CO- for a residue to the right of X= -CO-, or
-CO-CR-NH- for a residue to the left of X= -CO-;
wherein R is H- or an optionally substituted alkyl or alkylaryl group which may optionally include a hetero atom and may optionally be linked to the N atom.

10. The composition of any of claims 1-5 wherein the serine protease inhibitor is a borate or a boronic acid derivative having the formula B(OH)₂-C₆H₄-CO-R wherein -C₆H₄- has bonds attached in the *p*-position, and R is selected from the group consisting of hydrogen, hydroxy, C₁-C₆ alkyl substituted C₁-C₆ alkyl, C₁-C₆ alkenyl and substituted C₁-C₆ alkenyl, e.g. 4-formylphenyl-boronic acid (4-FPBA).

11. Use of a serine protease inhibitor to stabilize a serine hydrolase acting on ester bonds which is a lipase in the absence of a protease.

## Patentansprüche

1. Zusammensetzung, die eine an Esterbindungen wirkende Serinhydrolase, die eine Lipase ist, und einen Serinproteaseinhibitor umfasst, und die keine Protease umfasst, wobei die Zusammensetzung entweder eine Detergenszusammensetzung oder eine Nicht-Detergenszusammensetzung ist, wobei die Nicht-Detergenszusammensetzung die Lipase in einer Menge von 5-40 mg Enzymprotein pro g umfasst.

2. Zusammensetzung nach Anspruch 1, die in der Form von Körnchen oder einer Flüssigkeit ist.

3. Zusammensetzung nach Anspruch 1 oder 2, die eine Detergenszusammensetzung ist, die weiterhin ein oberflächenaktives Mittel umfasst.

4. Zusammensetzung nach Anspruch 1 oder 2, die kein oberflächenaktives Mittel umfasst.

5. Zusammensetzung nach einem beliebigen voranstehenden Anspruch, wobei die Lipase eine Aminosäuresequenz aufweist, die mindestens 80% identisch zur *Thermomyces lanuginosus*-Lipase, *Candida antarctica-Lipase* A oder *Candida antarctica-Lipase* B ist.

6. Zusammensetzung nach einem beliebigen voranstehenden Anspruch, wobei der Serinproteaseinhibitor die allgemeine Formel:
A⁴-A³-A²-A¹
aufweist, wobei
A¹ ein Aldehyd (CHO) oder Aldehydanalogon (COCX₃, COCHX₂, COCH₂X oder COCH₃, wobei X ein Halogenatom ist), ein Phosphorderivat oder eine Boronsäure (B(OH)₂) ist;
A² ein aromatischer oder ein aliphatischer Teil sein kann, wenn A² eine Aminosäure ist, dann ist der C-terminale Kohlenstoff im vorstehend beschriebenen A¹ eingeschlossen;
A³, wenn vorhanden, ein nicht sperriger Linker, bevorzugt hydrophob sein kann;
A⁴, wenn vorhanden, eine Schutzgruppe (z.B. eine wie nachstehend beschriebene N-capping-Gruppe), eine beliebige Aminosäure, eine kleine oder sperrige Gruppe sein kann.

7. Zusammensetzung nach einem beliebigen voranstehenden Anspruch, wobei der Serinproteaseinhibitor ein Peptidaldehyd ist.

8. Zusammensetzung nach einem beliebigen voranstehenden Anspruch, wobei das Peptidaldehyd die Formel
B²-B¹-B⁰-R
aufweist, wobei:
R Wasserstoff, CH₃, CX₃, CHX₂ oder CH₂X ist, wobei X ein Halogenatom ist;
B⁰ ein einzelner Aminosäurerest ist;
B¹ ein einzelner Aminosäurerest ist; und
B² aus einem oder mehreren Aminosäureresten (bevorzugt einem oder zwei) besteht, gegebenenfalls umfassend eine N-terminale Schutzgruppe.

9. Zusammensetzung nach einem beliebigen der Ansprüche 1-5, wobei der Serinproteaseinhibitor die Formel:
P-O-(Aⁱ-X')ₙ-Aⁿ⁺¹-Q
aufweist
wobei Q Wasserstoff, CH₃, CX₃, CHX₂ oder CH₂X ist, wobei X ein Halogenatom ist;
wobei ein X' die "doppelte N-capping-Gruppe" CO, CO-CO, CS, CS-CS oder CS-CO ist, am meisten bevorzugt Ureido/"Harnstoff" (CO), und die anderen X nichts sind;
wobei n = 1-10, bevorzugt 2-5, am stärksten bevorzugt 2;
wobei jedes von Aⁱ und Aⁿ⁺¹ ein Aminosäurerest mit der Struktur:
-NH-CR-CO- für einen Rest rechts von X= -CO-, oder
-CO-CR-NH- für einen Rest links von X= -CO- ist;
wobei R H- oder eine gegebenenfalls substituierte Alkyl- oder Alkylarylgruppe ist, die gegebenenfalls ein Heteroatom einschließen kann und gegebenenfalls mit dem N-Atom verknüpft sein kann.

10. Zusammensetzung nach einem beliebigen der Ansprüche 1-5, wobei der Serinproteaseinhibitor ein Borat oder ein Boronsäurederivat mit der Formel B(OH)₂-C₆H₄-CO-R ist, wobei -C₆H₄- Bindungen an der p-Position angefügt aufweist, und R ausgewählt ist aus der Gruppe bestehend aus Wasserstoff, Hydroxy, C₁-C₆-Alkyl-substituiertem C₁-C₆-Alkyl, C₁-C₆-Alkenyl und substituiertem C₁-C₆-Alkenyl, z.B. 4-Formylphenylboronsäure (4-FPBA).

11. Verwendung eines Serinproteaseinhibitors, um eine an Esterbindungen wirkende Serinhydrolase, die eine Lipase ist, in der Abwesenheit einer Protease zu stabilisieren.

## Revendications

1. Composition qui comprend une sérine hydrolase agissant sur les liaisons ester qui est une lipase et un inhibiteur de sérine protéase, et qui ne comprend pas de protéase, la composition étant soit une composition détergente soit une composition non détergente, la composition non détergente comprenant ladite lipase dans une quantité de 5 à 40 mg de protéine enzymatique par g.

2. Composition selon la revendication 1, qui est sous la forme de granulés ou de liquide.

3. Composition selon la revendication 1 ou 2, qui est une composition détergente comprenant en outre un tensioactif.

4. Composition selon la revendication 1 ou 2, qui ne comprend pas de tensioactif.

5. Composition selon l'une quelconque des revendications précédentes, dans laquelle la lipase a une séquence d'acides aminés qui est au moins identique à 80 % avec la lipase de *Thermomyces lanuginosus,* la lipase A de *Candida antarctica* ou la lipase B de *Candida antarctica*.

6. Composition selon l'une quelconque des revendications précédentes, dans laquelle l'inhibiteur de sérine protéase a la formule générale :
A⁴-A³-A²-A¹,
où
A¹ représente un aldéhyde (CHO) ou un analogue d'aldéhyde (COCX₃, COCHX₂, COCH₂X ou COCH₃, où X représente un atome d'halogène), un dérivé de phosphore ou un acide boronique (B(OH)₂) ;
A² peut représenter une partie aromatique, ou aliphatique, si A² est un acide aminé, alors le carbone C-terminal est inclus dans A¹ décrit ci-dessus ;
A³, si présent, peut représenter un lieur non volumineux, de préférence hydrophobe ;
A⁴, si présent, peut représenter un groupe protecteur (par exemple, un groupe N-coiffant tel que décrit ci-dessous), un acide aminé quelconque, un groupe petit ou volumineux.

7. Composition selon l'une quelconque des revendications précédentes, dans laquelle l'inhibiteur de sérine protéase est un aldéhyde peptidique.

8. Composition selon l'une quelconque des revendications précédentes, dans laquelle l'aldéhyde peptidique a la formule
B²-B¹-B⁰-R,
dans laquelle :
R représente un atome d'hydrogène, un groupe CH₃, CX₃, CHX₂, ou CH₂X, où X représente un atome d'halogène ;
B⁰ représente un résidu d'acide aminé unique ;
B¹ représente un résidu d'acide aminé unique ; et
B² est constitué d'un ou de plusieurs résidus d'acides aminés (de préférence un ou deux), comprenant éventuellement un groupe protecteur N-terminal.

9. Composition selon l'une quelconque des revendications 1 à 5, où l'inhibiteur de sérine protéase a la formule :
P-O-(Aⁱ-X')ₙ-Aⁿ⁺¹-Q
dans laquelle Q représente un atome d'hydrogène, un groupe CH₃, CX₃, CHX₂, ou CH₂X, où X représente un atome d'halogène ;
dans laquelle un X' représente le « groupe N-coiffant double » CO, CO-CO, CS, CS-CS ou CS-CO, de façon préférée entre toutes uréido/« urée » (CO) et les autres X' ne représentent rien ;
dans laquelle n = 1 à 10, de préférence 2 à 5, de façon préférée entre toutes 2 ;
dans laquelle chacun de Aⁱ et Aⁿ⁺¹ représente un résidu d'acide aminé ayant la structure :
-NH-CR-CO- pour un résidu à droite de X = -CO-, ou
-CO-CR-NH- pour un résidu à gauche de X = -CO- ;
où R représente H- ou un groupe alkyle ou alkylaryle éventuellement substitué qui peut comprendre éventuellement un hétéroatome et peut être éventuellement lié à l'atome N.

10. Composition selon l'une quelconque des revendications 1 à 5, dans laquelle l'inhibiteur de sérine protéase est un borate ou un dérivé d'acide boronique ayant la formule B(OH)₂-C₆H₄-CO-R dans laquelle -C₆H₄- a des liaisons fixées en position p, et R est choisi dans le groupe constitué d'un atome d'hydrogène, des groupes hydroxy, alkyle en C₁ à C₆ substitué par un groupe alkyle en C₁ à C₆, alcényle en C₁ à C₆ et alcényle en C₁ à C₆ substitué, par exemple, l'acide 4-formyl-phényl-boronique (4-FPBA).

11. Utilisation d'un inhibiteur de sérine protéase pour stabiliser une sérine hydrolase agissant sur les liaisons ester qui est une lipase en l'absence d'une protéase.
